# EUROPEAN PATENT SPECIFICATION

(11) **EP 3 986 345 B1**
(45) Date of publication and mention of the grant of the patent: **06.11.2024**
(21) Application number: 20809733.7
(22) Date of filing: 20.05.2020
(51) Int. Cl.: A61F 5/56, A42B 3/18, A61M 16/00, A61M 16/06, A61M 16/20

(54) **APPARATUS AND METHOD FOR DISRUPTING AND PREVENTING SNORE AND SLEEP APNEA**
VORRICHTUNG UND VERFAHREN ZUM UNTERBRECHEN UND VERHINDERN VON SCHNARCHEN UND SCHLAFAPNOE
APPAREIL ET PROCÉDÉ D'INTERRUPTION ET DE PRÉVENTION DE RONFLEMENT ET D'APNÉE DU SOMMEIL

(30) Priority: 21.05.2019 US 201962851074 P
(43) Date of publication of application: 27.04.2022
(73) Proprietor: Hariri, Sahar, Toronto ON M6K 2W2 (CA); Hariri, Aliasghar, Toronto, Ontario M2N 4L2 (CA)
(72) Inventor: HARIRI, Aliasghar, Toronto, Ontario M2N 4L2 (CA)
(74) Representative: Barker Brettell LLP
(86) International application number: PCT/CA2020/050677
(87) International publication number: WO 2020/232547

(56) References cited:
- WO-A1-2016/145510
- US-A1- 2016 089 261
- US-B2- 9 675 774
- US-B2- 9 937 312
- US-B2- 9 993 605

## Description

### FIELD OF THE INVENTION

The present invention relates to methods and apparatuses for disrupting and preventing snore and sleep apnea. Furthermore, the present invention relates to methods and apparatus for disrupting and preventing snore and sleep apnea, by means including slight movement of the head of a user and administration of positive air pressure.

### BACKGROUND OF THE INVENTION

A common reason for snoring is the vibration caused by the partial obstruction of the respiratory tract or airway due to the relaxation of the muscles when an individual is asleep. In some cases, this can extend to Obstructive Sleep Apnea (OSA), which is a more drastic or a full blockage of the respiratory tract or airway. Statistically, at least 30% of adults snore and this number is 60% for men and 40% for women aged 60 to 65, suggesting a positive correlation between aging and susceptibility to snoring. Snoring is known to cause sleep deprivation both for the snorers and for the individuals around them, resulting in daytime drowsiness, irritability lack of focus, and reduced libido. It has also been suggested that snoring can cause significant psychological and social damages and burdens for snorers. Multiple studies reveal a positive correlation between snoring and increased risk of heart attack by about 34% and increased risk of stroke by about 67%. In some cases, snore can develop into Obstructive Sleep Apnea (OSA), which is a more drastic or full blockage of the respiratory tract or airway with more serious associated health risks.

There are many devices for the purpose of alleviating snore or sleep apnea. Some of these devices relate to pillows that either fix or passively or actively change the position of the head of the user, as presented in U.S. Pat. No. 7,676,870B2 and PCT/EP2012/072923 applications. The pillow designs that change the position of the user, require detection of the position of the head to overcome the limitation of an isolated displacement component and further limit the pillow choice of the user. Wearable devices that stimulate a user by sound, vibration, or electric shock, as presented in U.S. Pat. No 5,477,867 may be invasive. There are further positive airway pressure devices used to disrupt or prevent sleep apnea (may also be used for snore but not done so conventionally), which use positive air pressure to in part open the blockage of the respiratory tract or airway. Inhalation and more so exhalation in a positive air pressure can be uncomfortable for the user and disrupt the sleep and comfort of the user. WO2016145510 discloses a CPAP system with a cervical collar with an inflatable element which can help keep the mouth of the patient closed during treatment. The cervical collar is not worn around the head of the user and does not cause the head to move as such.

### SUMMARY OF THE INVENTION

The invention is defined in claim 1 and its appended dependent claims.

In accordance with one aspect of the disclosure, the apparatus comprises an inflatable headgear that is configured to be inflated by an air inflator through a tube and deflated through a release valve. The air inflator is actuated by the control commands send to a control unit from a processing unit, upon detection of a trigger event by means of a sensor. The movement caused by the inflation and deflation of the inflatable headgear may move the head of a user and disrupt or prevent snore or sleep apnea.

In accordance with another aspect of the disclosure, the apparatus further comprises a breathing mask held in place by the inflatable headgear, and a positive airway pressure deceive which is configured to administrate positive air pressure through a flexible hose and the breathing mask, when actuated by the control commands sent to the control unit from the processing unit. The apparatus may use one or both of, the movement caused by the inflation and deflation if the inflatable headgear and the positive air pressure administrated by the positive airway pressure device to interrupt or prevent snore and sleep apnea.

In accordance with yet another aspect of the disclosure, the apparatus comprises a breathing mask held in place by a headgear, an active atmospheric or ambient valve, a sensor for detection of a trigger event, a positive airway pressure device configured to administrate positive air pressure though a flexible hose and the breathing mask when actuated by the control commands sent to the control unit from the processing unit. The control unit is configured to turn on and off the positive airway pressure device and open and close the active atmospheric or ambient valve based on the control commands sent by the processing unit. The active atmospheric or ambient valve allows for comfortable breathing without a positive air pressure during the absence of a trigger event that may relate to snore and sleep apnea.

In accordance with a further aspect of the disclosure, the headgear of the apparatus comprises an inflatable headgear, and the apparatus further comprises an air inflator configured to inflate the inflatable headgear though a tube, a release valve configured to allow for the deflation of the inflatable headgear, wherein the processing unit is configure to transmit control commands to the control unit to turn on and off the air inflator, and open and close the release valve. The apparatus may use one or both of the, movement caused by the inflation and deflation of the inflatable headgear and the positive air pressure administrated by the positive airway pressure device to interrupt or prevent snore and sleep apnea. This is while the active atmospheric or ambient valve allows for comfortable breathing without a positive air pressure during the absence of a trigger event that may relate to snore and sleep apnea.

In accordance with yet another aspect of the disclosure, the active atmospheric or ambient valve, as discussed in the above aspects, can be replaced by a passive atmospheric or ambient valve or any other type of valve that would enable the intended function. In case of a passive valve, the valve will be a normally open valve when there is no administration of positive air pressure. The valve closes as the result of the pressure buildup in the mask when the administration of positive air pressure begins, and is opened again, when the positive air pressure device stops administration of positive air pressure and the pressure in the breathing mask is dropped to the ambient pressure.

It is an object of the present invention to provide a novel apparatus for disrupting or preventing snoring and sleep apnea where in one embodiment this is done by slight movement of the head of a user, using an inflatable headgear, which does not require a pillow system, or the detection of the location of the head and is further non-invasive, therefore improving the method of disruption and prevention of snore and sleep apnea by movement. In accordance in one aspect of this invention, the displacement component is worn around the head of the user and therefore the position of the head of the user does not need to be detected and is not a concern. The present invention further does not comprise a pillow and is not required to work in conjunction with a pillow and therefore can accommodate pillow option or lack of use of a pillow, making it convenient to use and portable. The use of the inflatable headgear in conjunction with the positive airway pressure device can further reduce the time required to administrate pressurized air and allow for a more comfortable sleep. In detecting and disrupting snore, this invention reduces the possibility and frequency of the user's occurrence of sleep apnea and the need for positive air pressure administration of the positive airway pressure device, resulting in a more comfortable sleep. The present invention is further meant to address the discomfort in using positive air pressure devices for addressing sleep apnea which include tolerating the pressurized air administrated by the positive air pressure device, claustrophobia resulting from breathing in a closed breathing mask, and difficulty falling asleep due to lack of comfort. In doing so, the present invention allows for open ambient pressure breathing in the absence of snore, sleep apnea or other trigger events, enabling the user to fall asleep in ambient pressure, breathing directly into the room, and in doing so eliminates/reduces the experience of claustrophobia when the user is falling asleep or any anytime during the use when positive air pressure administration is not required. The present invention further allowed for delaying and reducing the duration of snore or sleep apnea breathing events throughout the night and therefore reducing the duration of high air pressure administration. The present invention further reduces the severity of snore, sleep apnea and other respiratory events and therefore reducing the required administered air pressure by the positive air pressure device. The passive atmospheric or ambient valve does not require to be in communication with the control unit as it functions based on the pressure difference inside the mask an in the ambient space.

### BRIEF DESCRIPTION OF THE DRAWINGS

A detailed description of the preferred embodiments is provided herein below by way of example only and with reference to the following drawings, in which:

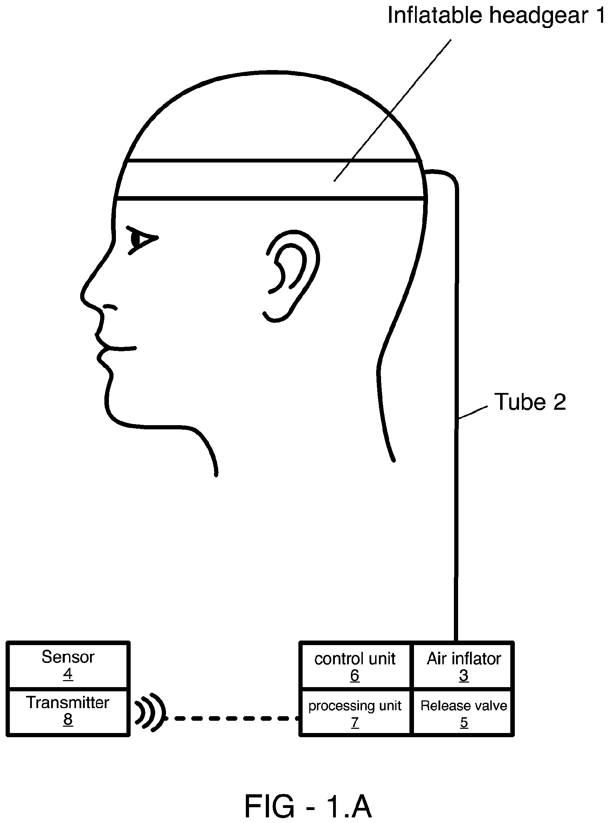

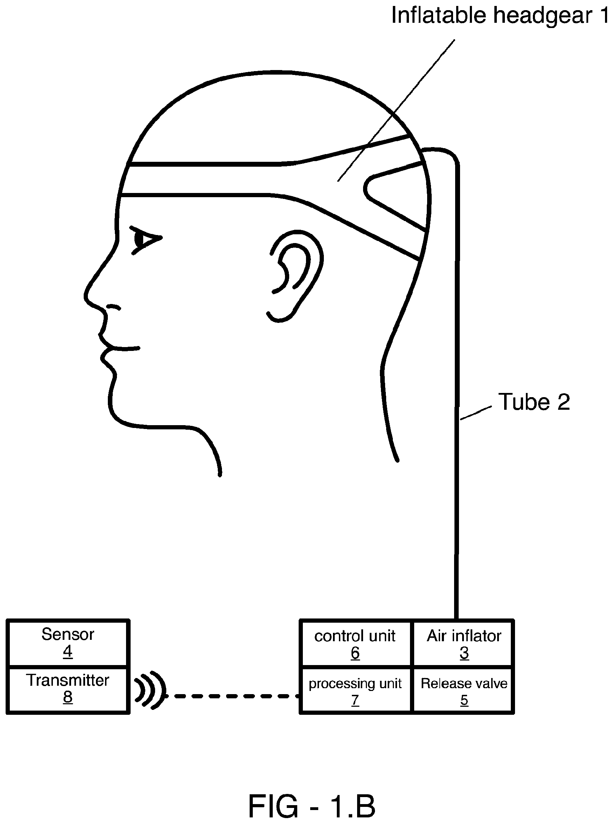

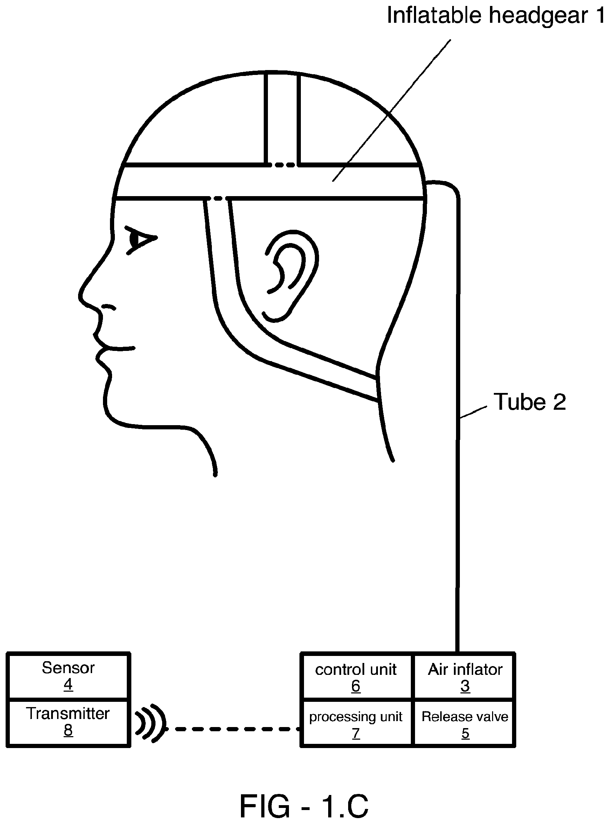

FIG.1.A is an illustration of a preferred embodiment of the present invention, wherein the inflatable strap assembly of the inflatable headgear comprises one inflatable strap.
FIG.1.B is an illustration of a preferred embodiment of the present invention, wherein the inflatable strap assembly of the inflatable headgear comprises more than one inflatable strap.
FIG.1.C is an illustration of yet another preferred embodiment of the present invention, wherein the inflatable strap assembly of the inflatable headgear comprises more than one inflatable strap.
FIG.2.A is an illustration of an embodiment of the present invention, wherein the inflatable headgear is in a deflated position.

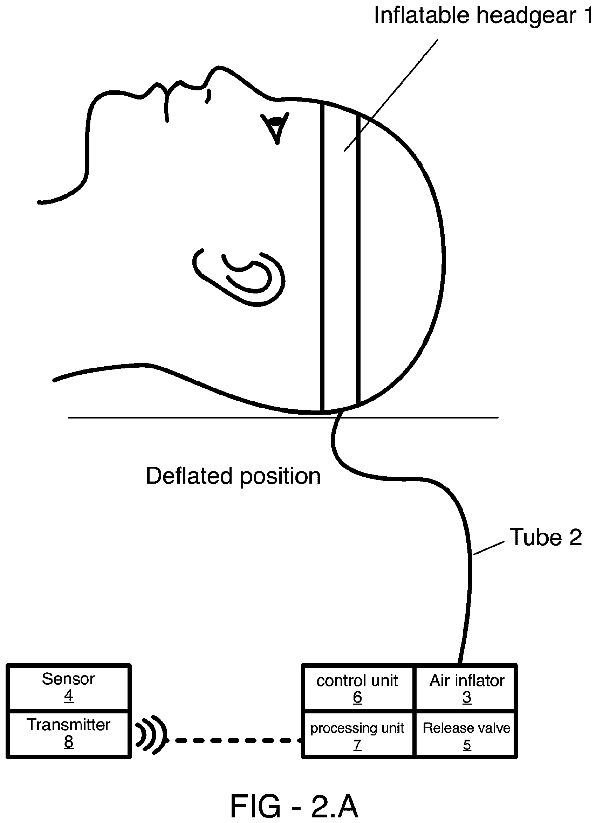

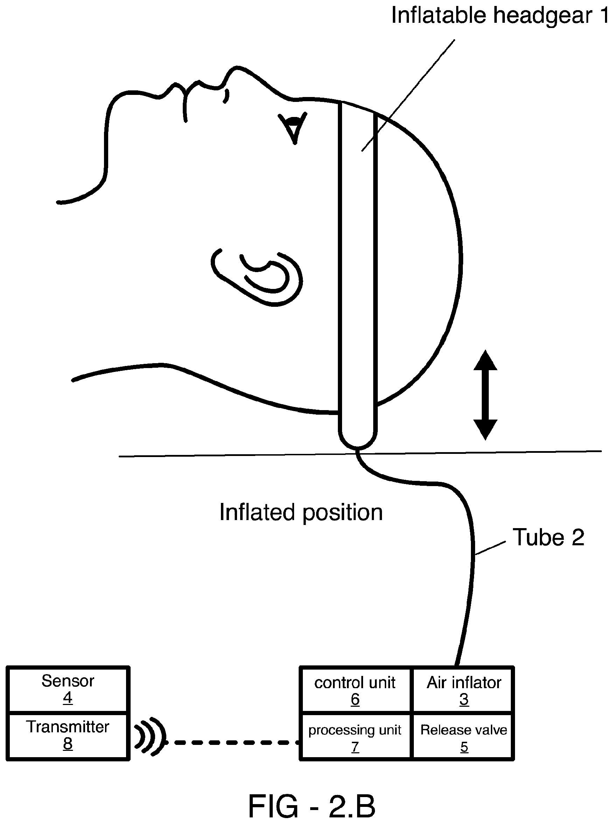

FIG.2.B is an illustration of an embodiment of the present invention, wherein the inflatable headgear is in an inflated position.

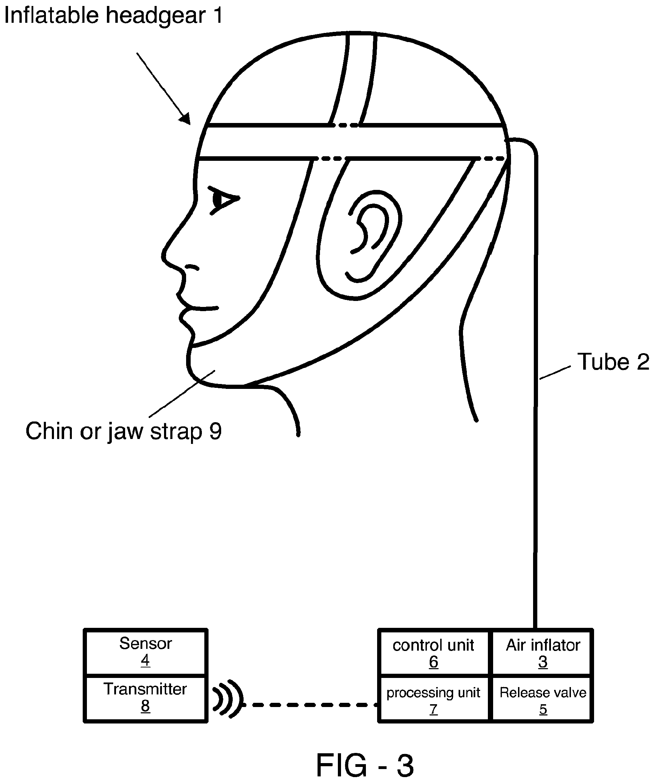

FIG.3 is an illustration of an embodiment of the present invention, wherein the inflatable headgear comprises a chin or jaw strap.

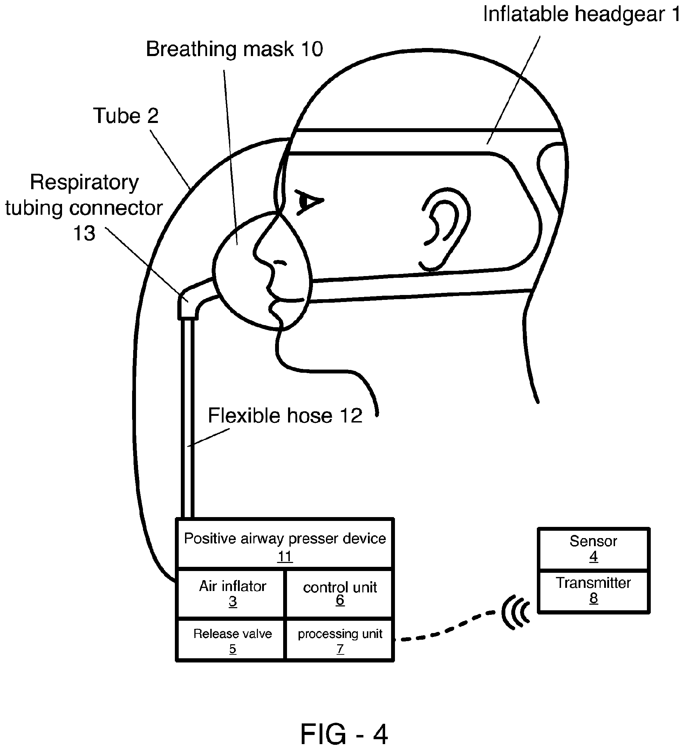

FIG.4 is an illustration of another embodiment of the present invention, wherein an inflatable headgear is used for movement while a positive airway pressure device is used for administration of positive air pressure.

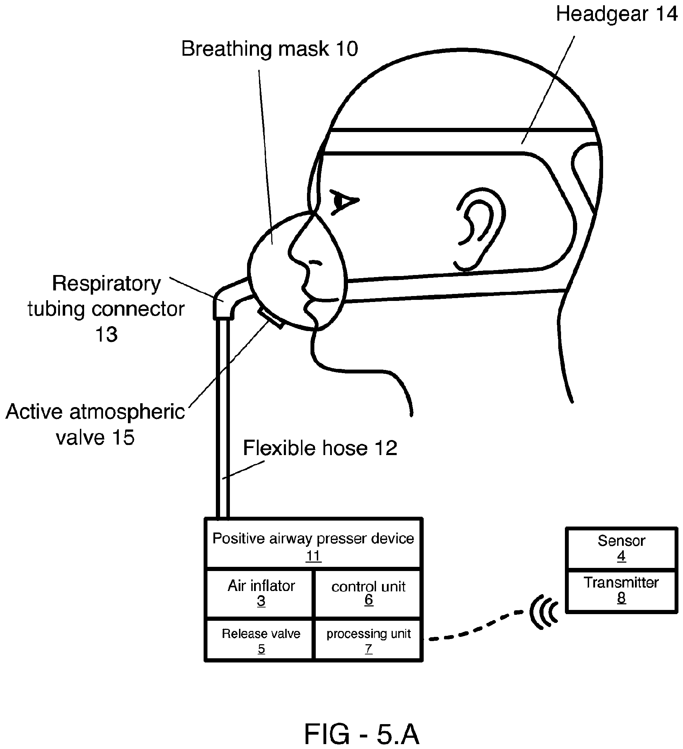

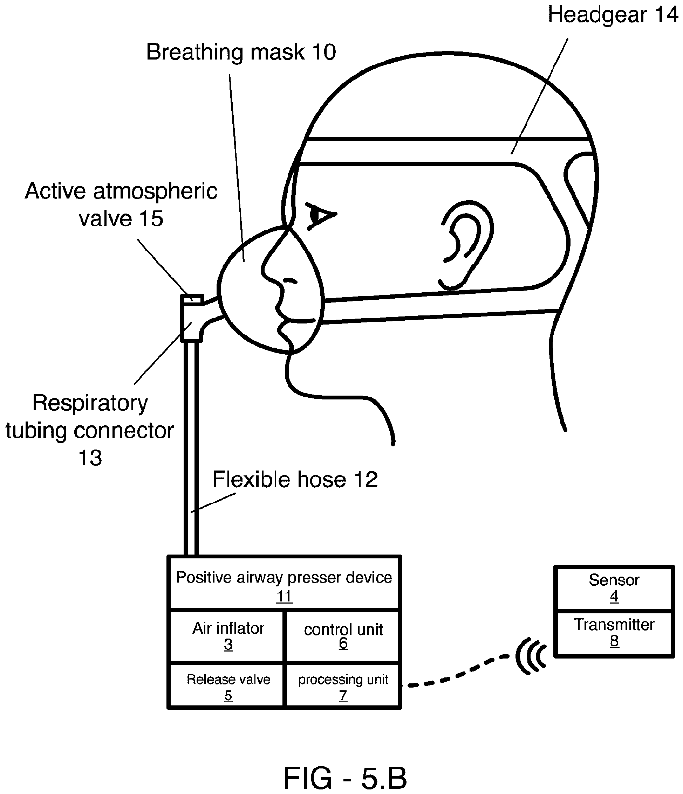

FIG.5.A is an illustration of another embodiment of the present invention, wherein a positive airway pressure device is used in conjunction with an active atmospheric or ambient valve, wherein the breathing mask comprises an active atmospheric or ambient valve.
FIG.5.B is an illustration of another embodiment of the present invention, wherein a positive airway pressure device is used in conjunction with an active atmospheric or ambient valve, wherein the respiratory tubing connector comprises an active atmospheric or ambient valve.

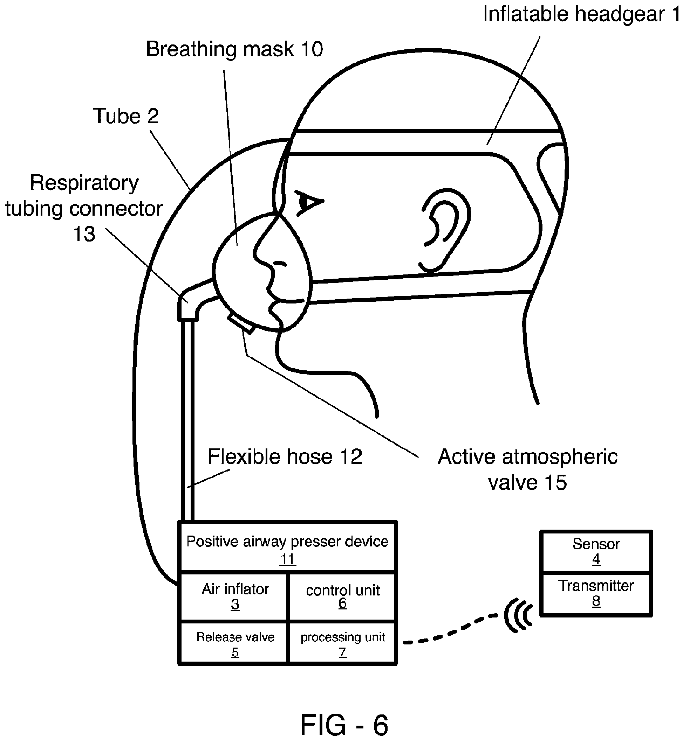

FIG.6 is an illustration of another embodiment of the present invention, wherein an inflatable headgear is used for movement while a positive airway pressure device is used for administration of positive air pressure and is used in conjunction with an active atmospheric or ambient valve.

### DETAILED DESPCITION OF THE INVENTION

FIG.1A is a schematic drawing of an embodiment of the apparatus. The apparatus may comprise an inflatable headgear 1, a tube 2 connecting at least one air inflator 3 to the inflatable headgear 1, wherein the inflatable headgear is configured to inflate and expand, and deflate and collapse, at least one sensor 4 for detecting a trigger event, at least one release valve 5 configured to deflate the inflatable headgear 1, a control unit 6 in communication with the air inflator 3 to actuate the air inflator 3 on to inflate the inflatable headgear 1, and the control unit 6 further in communication with the release valve 5 to close the release valve 5 during the inflation of the inflatable headgear 1 and open the release valve 5 during the deflation of the inflatable headgear 1, a processing unit 7 in communication with the control unit 6, the processing unit 7 comprising at least one processor(not shown) and a memory storing instructions which when executed by the processor configures the processor to detect a trigger event, and the processor to configured to transmit control commands to the control unit 6 to turn on and off the air inflator 3 and open and close the release valve 5.

The sensor 4 may be coupled with a wired or wireless transmitter 8, wherein the transmitter 8 is configured to transmit the data from the sensor 4 to the processing unit 7. The sensor 4 can be audio sensor or microphone. The apparatus may further comprise at lest one silencer (not shown) to reduce the noise and vibration of the air inflator 3. FIG.1.A, FIG.1.B and FIG.1.C show example embodiments of the inflatable headgear 1 which may comprise an inflatable strap assembly comprising at least one inflatable strap. FIG1.A shows an embodiment of the inflatable headgear 1 comprising only one inflatable strap, wherein FIG.1.B and FIG.1.C show example embodiments of the inflatable headgear 1 comprising an inflatable strap assembly with at least one inflatable strap, and in these cases more than one inflatable strap. As shown in FIG.1.A, FIG.1.B, and FIG.1.C the inflatable headgear 1 comprises an inflatable strap assembly, which is configured to extend to the sides and the back of the head of the user. The inflatable headgear 1 may also be configured to extend to a forehead, crown of the head, or the neck of a user. At least one strap that forms the inflatable headgear 1 may comprise at least one inflatable chamber (not shown) positioned inside a strap, where the strap can be larger than the inflatable chamber in width or made of stretchable material to allow for the expansion of the inflatable chamber. The inflatable chamber may be longitudinal and have the shape of a tube. The inflatable chamber can extend to a part or the entirety of the length of the strap. The inflatable strap can be made of any stretchable or non-searchable material, including but nor limited to plastic and fabric. The inflatable strap can further comprise an inflatable chamber, where the inflatable chamber is the inflatable strap, and is being used instead of or in place of the inflatable strap. The tube 2 connecting the inflatable headgear 1 to the air inflator 3 can be connected to the inflatable headgear 1 anywhere on the inflatable headgear 1 including the back or the front of the inflatable headgear 1. The inflatable headgear 1 comprises an inflatable strap assembly, wherein the inflatable strap assembly comprises at least one inflatable strap, the inflatable strap assembly may further comprise at least one non-inflatable strap. The inflatable strap may further be combined with non-inflatable straps comprising a strap with inflatable and non-inflatable sections.

FIG.2.A and FIG.2.B show an embodiment of the inflatable headgear 1 in deflated and inflated positions. FIG.2.A shows the inflatable headgear 1 in a deflated position, prior to inflation or after deflation. FIG.2.B shows the inflatable headgear 1 in an inflated position, wherein the inflation and the expansion of one or more of the inflatable straps of the inflatable strap assembly of the inflatable headgear 1 cause the head of a user to move. The inflatable headgear 1 is configured to inflate and expand, and deflate and collapse, while supporting the head of a user. An inflation and deflation cycle comprises an inflation of the inflatable headgear 1 by the air inflator 3 when actuated by the control unit 6 upon detection of a trigger event, followed by a deflation of the inflatable headgear 1 through the release valve 5 when the control unit 6 signals the opening of the release valve 5. The inflation and deflation cycle can comprise the inflatable headgear 1 starting from an initial position of deflation and returning back to the initial position after a cycle of inflation and deflation. The deflation following the inflation can be immediate or can include a time delay. The inflation and deflation of the inflatable headgear 1 each may also occur in multiple steps of inflations and deflations. The inflation and deflation cycle may be configured by timing where in response to the trigger event, the control unit 6 may be configured to actuate the air inflator 3 on with an inflating time and subsequently off with a deflating time, wherein the inflating time and deflating time comprise an inflation deflation cycle. The inflation and deflation cycle may further be configured by pressure, wherein in response to the trigger event, the control unit 6 is configured to actuate the air inflator 3 on to inflate the inflatable headgear 1 until the control unit 6 reaches a pre-determined pressure and subsequently actuate the air inflator 3 off until the control unit 6 reaches a deflation pressure, wherein the inflation and deflation comprise a cycle. The inflation of the inflatable headgear 1 may be configured by speed, wherein in the control unit 6 is configured to control a speed at which the air inflator 3 inflates the inflatable headgear 1 at a pre-determined inflation rate to provide different inflation patterns. The inflation of the inflatable headgear 1 may further be configured by size of the expansion of the inflatable headgear 1, wherein the control unit 6 is configured to actuate the air inflator 3 on to inflate the inflatable headgear 1 until the inflatable headgear 1 reaches a predetermined inflation size.

The processing unit 7 may further be coupled to at least one sensor 4 which may be an audio sensor or microphone and be configured to receive sound waves from a plurality of sources as detected by the one or more audio sensors or microphones, and wherein the processing unit 7 is configured to identify a snore or sleep apnea sound source by analyzing sound waves, to recognize and identify a sound of snore or sleep apnea among plurality of sounds and to also recognize a sound of the snore or sleep apnea of a user of the apparatus among plurality of snore or sleep apnea sounds in case there are multiple cases of snore or sleep apnea in a proximity. The processing unit 7 may further be coupled to two or more sensors that can be audio sensors or microphones, and is further configured to receive sound waves from a plurality of sources as detected by two or more audio sensors or microphones, and wherein the processing unit 7 may be configured to identify a location of a snore or sleep apnea sound source by analyzing the sound waves. The processing unit 7 may use triangulation to identify the location of a user of the apparatus among plurality of snore and sleep apnea sounds, and therefore react only to the snore or sleep apnea of the user of the apparatus.

A trigger event may comprise detection of a snore, or detection of sleep apnea, or identification of a snore or sleep apnea or a snore or sleep apnea source, or prediction of occurrence of snore or sleep apnea. The trigger event may also comprise detection, identification or prediction of a snore or sleep apnea event or an indication of snore or sleep apnea. The detection of a snore or sleep apnea can be done by an audio sensor or microphone that would detect the snore or sleep apnea sound. It can also be done by other sensors, for example detecting vibration of the throat causes by a snore or sleep apnea, or a sensor that would detect snore or sleep apnea from the oxygen level in the blood of a user or other biological parameters and markers that can identify or predict the occurrence of snore or sleep apnea. The trigger event can further comprise one or more of the said events. The control unit 6 may further be configured to initiate inflation and deflation cycles based on a predetermined time interval, independent of the trigger event.

The inflatable headgear may further comprise at least one light, at least one vibrating component, and at least one speaker to use light, vibration, and sound as other means of stimulation of the user to stop or prevent snore and sleep apnea. These components will be in communication with the control unit. These components can be used in combination or separately. For effective use of the light, the inflatable headgear 1 may further comprise flappers that may cover the eye and act as eye mask, wherein the lights may be placed under the flappers for close approximation to the eyes. The lights may further be utilized for alternative functions such as being configured to also function as wake-up light alarm clock. The speakers may be integrated as part of the inflatable headgear and may be located close to the ears of a user. The inflatable headgear 1 may further comprise flaps that cover the ears where the speakers may be placed in.

FIG.3 shows an embodiment of the apparatus wherein the inflatable headgear 1, comprising an inflatable strap assembly, further comprises a chin or jaw strap 9 as part of the inflatable strap assembly. The sections of the chin or jaw strap that extend to the sides of the face, the jaw and the chin of a user may be configured not to be inflatable and function to hold the chin or jaw in place and pull the chin inwards. The chin or jaw strap 9 may be configured to look and function similar to conventional jaw straps or chin straps used to stop and prevent snore and sleep apnea.

FIG.4 shows an embodiment of the apparatus wherein the said apparatus further comprises a breathing mask 10, where the inflatable headgear 1 is further configured to connect to the breathing mask 10 and keep it in place in accordance with its use, a positive airway pressure device 11, a flexible hose 12 connecting the breathing mask 10 to the positive airway pressure device 11, wherein the control unit 6 is further configured to be in communication with the positive airway pressure device 11 in addition to being in communication with the air inflator 3 and the release valve 5, wherein the control unit 6 may turn on and off the positive airway pressure device 11, actuate the positive airway pressure device 11 to administrate, and increase and decrease the pressure of the administrated positive air pressure, wherein the processing unit 7 is configured to detect trigger event and transmit control commands to the control unit 6 to trigger actuation of at least one of the air inflator 3 or the positive airway pressure device 11. The breathing mask 10 may be any respirator breathing mask, including but not limited to breathing masks that are nasal and cover the nose, cover nose and mouth, or fullface masks. The inflatable headgear 1 shown in FIG.4 and the remaining figures is yet another embodiment of the inflatable strap assembly configured in accordance with the previously said specifications as well as being configured to hold a breathing mask 10 in place. The breathing mask 10 may be connected to the flexible hose 12 by means of a respiratory tubing connector 13.

The positive airway pressure device 11 may comprise at least one blower (not shown) to supply positive air pressure. The positive airway pressure device 11 may further comprise at least one air filter (not shown), at least one humidifier (not shown), and at least one heater (not shown).

The positive airway pressure device 11 might further comprise at least one silencer to reduce the vibration and sound of the blower. The positive airway pressure device 11 is configured to pressurize air and create positive air pressure, which is administrated through the breathing mask 10. The positive air pressure may in part open the blockage of the respiratory tract or airway that may results in snore or sleep apnea, and therefore may alleviate snore or sleep apnea.

Upon the detection of a trigger even by the processing unit 7, the control unit 6 is configured to actuate the air inflator 3 on to inflate the inflatable headgear 1. The control unit 6 is further configured to close the release valve 5 upon detection of the trigger event. The inflation is followed by a deflation as the control unit 6 turns off the air inflator 3 and opens the release valve 5 allowing for deflation of the inflatable headgear 1 and its returning to the initial deflated position. The trigger event may be related to snore or sleep apnea. Examples of the trigger event would be the sound of snore detected by an audio sensor of microphone or detection of sleep apnea from the pattern of breathing indicating occurrence of sleep apnea detected by at least one appropriate sensor that may be positioned inside the breathing mask 10 or at the other end of the flexible hose 12, or anywhere on or off the apparatus. An example may be sound of snore or sleep apnea detected by an audio processor, which triggers the inflation of the inflatable headgear 1 followed by its deflation. If the snore or sleep apnea is not stopped, a trigger event will be detected, and an inflation and deflation cycle will take place. The inflation and deflation cycles will continue as long as the processing unit 7 detects a trigger event, which means that the snore or sleep apnea have not ceased yet. The inflation and deflation of the inflatable headgear 1 move the head of a user and may cause the stopping of the snore. This function of the apparatus, in this example, may enabled the stopping of a snore and preventing it from transforming into sleep apnea, and therefore preventing the need to use administration of positive air pressure by the positive airway pressure device 11.

In accordance with one aspect of the invention, upon detection of a trigger event by the processing unit 7, the control unit 6 is configured to actuate the positive airway pressure device 11 to initiate the administration of positive air pressure or increase the pressure of the administrated positive air pressure, wherein the positive air pressure is administrated through the breathing mask 10. The administrated positive air pressure may in part open the blockage of the respiratory tract or airway that may result in snore or sleep apnea, and therefore may alleviate snore or sleep apnea. The trigger even may be related to snore or sleep apnea. Examples of the trigger event would be the sound of snore detected by an audio sensor of microphone or detection of sleep apnea from the pattern of breathing indicating occurrence of sleep apnea detected by at least one appropriate sensor that may be positioned inside the breathing mask 10 or at the other end of the flexible hose 12, or anywhere on or off the apparatus. Therefore, upon a trigger event which can relate to snore or sleep apnea, the positive airway pressure device 11 starts the administration of or increases the pressure of positive air pressure that is administrated through the breathing mask 10, that may open the blockage of, and further stimulate, the respiratory tract or airway system and stop the snore or sleep apnea.

In accordance with another aspect of the invention, the increase in the positive air pressure of the positive airway pressure device 11 as actuated by the control unit 6 may be configured to take place in installments or multiple steps, wherein there may be a time period between each step or increment, where the positive air pressure is increased by another step if the trigger event does not cease. As example, the positive airway pressure device 11 may be set at an initial pressure of 4 cmHzO to start, upon detection of a trigger event, the control unit 6 may actuate the positive airway pressure device 11 to increase the positive air pressure to 10 cmHzO and stay at that pressure for a certain period of time, if after the said period of time a trigger event is detected, the control unit 6 may actuate the positive airway pressure device 11 to increase the positive air pressure to 15 cmHzO and stay at that pressure for a certain period of time, if after the said period of time a trigger event is detected, the control unit 6 may actuate the positive airway pressure device 11 to increase the positive air pressure to 20 cmHzO. The step increase of the positive air pressure may continue for set increments and set time intervals until the positive air pressure reaches the maximum pressure prescribed for a user. The numbers of steps, the pressure increases per steps, and the time intervals may vary and be predetermined or identified by algorithms based on the ongoing condition of the user. After a predetermined time during which the processing unit 7 does not detect a trigger event, which may mean the cessation of the trigger event, the control unit 6 is configured to signal the positive airway pressure device 11 to reduce the pressure of the positive air pressure or to stop the administration of positive air pressure. The reduction in the positive air pressure may take place in installments and multiple steps, where there may be a set-period of time between each step. As example, the positive airway pressure device 11 may be at a positive air pressure of 20 cmHzO when the cessation of a trigger event has been identified by the processing unit 7, upon the detection of the cessation of a trigger event the control unit 6 may actuate the positive airway pressure device 11 to reduce the positive air pressure to 15 cmHzO and stay at that pressure for a certain period of time, if after a pre-set period of time no trigger event is detected, the control unit 6 may actuate the positive airway pressure device 11 to reduce the positive air pressure to 10 cmHzO and stay at that pressure for a pre-set period of time, if after the said period of time a no trigger event is detected, the control unit 6 may actuate the positive airway pressure device 11 to reduce the positive air pressure to the initial 4 cmHzO. At any point during the reduction of the positive air pressure if the processing unit 7 detects a trigger event, the control unit 6 may actuate the positive airway pressure device 11 to increase the positive air pressure in ways that have been explained above. At least one sensor is continuously active to detect occurrence of trigger event including occurrence of snore or sleep apnea, to ensure that trigger events do not go unnoticed at any time.

In accordance with yet another aspect of the invention, in response to a trigger event, the control unit 6 may be configured to actuate the air inflator 3 on to inflate the inflatable headgear 1, and close the release valve 5, and also actuate the positive airway pressure device 11 to initiate the administration of positive air pressure or increase the pressure of positive air pressure administrated through the breathing mask 10. The increase of the positive air pressure can take place in instalments and multiple steps as explained previously. The control unit 6 may be configured to actuate the positive airway pressure device 11 to start the administration of positive air pressure or increase the positive air pressure after a predetermined time interval from the detection of the trigger event, while the control unit 6 may be configured to actuate the air inflator 3 immediately upon the detection of a trigger event by the processing unit 7. This will create a delay time between the actuation of the air inflator 3 and the actuation of the positive airway pressure device 11, during which the movements cause by the inflation or the inflation and deflation cycles of the inflatable headgear 1 may stop the snore or sleep apnea and therefore prevent the need of administration of or increase of the pressure of positive air pressure by the positive airway pressure device 11. The control unit 6 may be configures to initiate inflation and deflation cycles of the inflatable headgear 1 at a predetermined interval for a duration of the time during which the presence of a trigger event is detected.

In one aspect of the invention, the breathing mask 10 can be attached to and held in place by a non-inflatable headgear (not shown), to make the use of the breathing mask 10 and the inflatable headgear 1 independent of each other. For this embodiment, the apparatus will be configured so that the air inflator 3 and the positive airway pressure device 11 can each be turned off when the user wants to use only one of the two main functions of the apparatus, either the movement by the inflatable headgear 1 or the administration of positive air pressure by the positive airway pressure device 11.

FIG.5.A is an illustration of an embodiment of the apparatus comprising a breathing mask 10, a headgear 14 configured to hold the breathing mask in place, at least one active atmospheric or ambient valve 15, a positive airway pressure device 11, a flexible hose 12 connecting the breathing mask 10 to the positive airway pressure device 11, at least one sensor 4 for detecting a trigger event, a control unit 6 in communication with the positive airway pressure device 11 to turn on and off the positive airway pressure device 11, or to increase and decrease the pressure of the positive air pressure administrated by the positive airway pressure device 11, the control unit 6 further in communication with the active atmospheric or ambient valve 15 to open and close the active atmospheric or ambient valve 15, a processing unit 7 in communication with the control unit 6, the processing unit 7 comprising at least one processor(not shown) and a memory storing instructions which when executed by the processor configured the processor to detect a trigger event, and configures the processor to transmit control commands to the control unit 6. The breathing mask 10 may be connected to the flexible hose 12 by means of a respiratory tubing connector 13.

The active atmospheric or ambient valve 15 can comprise any type of valve that can be in communication with the control unit 6 to receive open and close commands. The use of active valve here is in contrast with a passive valve which may open and close as the result of gravitational force or pressure difference in the two sides of the valve. The said active atmospheric or ambient valve 15 can be integrated as part of the breathing mask 10 as shown in FIG.5.A or integrated as part of the respiratory tubing connector 13 that connects breathing mask 10 and the flexible hose 12 as shown in the FIG.5.B, or it may further be integrated as part of the flexible hose 12 (not shown).

The sensor 4 may be coupled with a wired or wireless transmitter 8, wherein the transmitter 8 is configured to transmit the data from the sensor 4 to the processing unit 7. The sensor 4 can be audio sensor or microphone.

The positive airway pressure device 11 may comprise at least one blower (not shown) to supply positive air pressure. The positive airway pressure device 11 may further comprise at least one air filter (not shown), at least one humidifier (not shown), and at least one heater (not shown). The positive airway pressure device 11 might further comprise at least one silencer to reduce the vibration and sound of the blower. The positive airway pressure device 11 is configured to pressurize air and create positive air pressure, which is administrated through the breathing mask 10. The positive air pressure may in part open the blockage of or stimulate the respiratory tract or airway, and therefore may alleviate snore or sleep apnea.

The processing unit 7 may further be coupled with at least one sensor 4 which may be an audio sensor or microphone and be configured to receive sound waves from a plurality of sources as detected by the one or more audio sensors or microphones, and wherein the processing unit 7 is configured to identify a snore or sleep apnea sound source by analyzing sound waves, to recognize and identify a sound of snore or sleep apnea among plurality of sounds and to also recognize a sound of the snore or sleep apnea of a user of the apparatus among plurality of snore or sleep apnea sounds in case there are multiple cases of snore or sleep apnea in a proximity. The processing unit 7 may further be coupled to two or more sensors that can be audio sensors or microphones, and is further configured to receive sound waves from a plurality of sources as detected by two or more audio sensors or microphones, and wherein the processing unit 7 may be configured to identify a location of a snore or sleep apnea sound source by analyzing the sound waves. The processing unit 7 may use triangulation to identify the location of a user of the apparatus among plurality of snore and sleep apnea sounds, and therefore react only to the snore or sleep apnea of the user of the apparatus.

The trigger event may comprise detection of a snore, or detection of sleep apnea, or identification of a snore or sleep apnea or a snore or sleep apnea source, or prediction of occurrence of snore or sleep apnea. The trigger event may also comprise detection, identification or prediction of a snore or sleep apnea event or an indication of snore or sleep apnea. The detection of a snore or sleep apnea can be done by an audio sensor or microphone that would detect the snore or sleep apnea sound. It can also be done by other sensors, for example detecting vibration of the throat causes by a snore or sleep apnea, or a sensor that would detect snore or sleep apnea from the oxygen level in the blood of a user or other biological parameters and markers that can identify occurrence of snore or sleep apnea. The trigger event can further comprise one or more of the said events.

In accordance with one aspect of the invention, the control unit 6 may be configured to turn on and increase the positive air pressure of the positive airway pressure device 11 and close the active atmospheric or ambient valve 15, followed by reducing the positive air pressure of and turning off the positive airway pressure device 11 and opening the active atmospheric or ambient valve 15, based on a predetermined time interval and independent of the trigger event. The control unit 6 may be further configured to increase and decrease the positive air pressure of the positive airway pressure device, base on a predetermined time interval and independent of the trigger event.

In accordance with one aspect of the invention, during the absence of a trigger event, the control unit 6 is configured to signal the active atmospheric or ambient valve 15 to remain open and also to signal the positive airway pressure device 11 to remain inactive and not administrate positive air pressure. Absence of a trigger event may comprise a predetermined time interval during which the processing unit does not detect a trigger event, which may indicate the absence of snore or sleep apnea. The time interval may be more relevant to snore due to its discontinuous pattern. During the absence of a trigger event, the active atmospheric or ambient valve 15 is open, while the positive airway pressure device 11 is inactive and does not administrate positive air pressure, allowing the pressure inside the breathing mask 10 to remain atmospheric or the same as the air pressure in the environment of the user, further allowing the user to breath at atmospheric or ambient pressure. The position of the active atmospheric or ambient valve 15 and its approximate closeness to the mouth and nose of a user, would open the airflow of the breathing mask 10 to the outside environment, allowing the user to breath comfortably, without additional pressure or the need to breath in a closed respiratory system, making breathing much more comfortable. When the processing unit 7 detects a trigger event, in response to the trigger event, the control unit 6 is configured to signal the actuation of the positive airway pressure device 11 and turn it on for the positive airway pressure device 11 to administrate positive air pressure through the breathing mask, followed by the control unit 6 actuating and closing the active atmospheric or ambient valve 15, allowing the positive air pressure inside the flexible hose 12 and the breathing mask 10 to build up an increase. The positive airway pressure device 11 may increase the positive air pressure in steps and intervals to a pre-determined maximum prescribed pressure as per the commands of the processing unit 7. If the processing unit 7 does not detect a trigger event after a predetermined period of time, indicating that a snore or sleep apnea has stopped, the control unit 6 signals the positive airway pressure device 11 to reduce the pressure of the positive air pressure. The reduction in pressure may take place in intervals and steps. When the pressure of the positive airway pressure device 11 reaches atmospheric or ambient, or near-atmospheric or ambient pressure, the control unit 6 signals the opening of the active atmospheric or ambient valve 15, allowing a user to breath in an atmospheric or ambient pressure and without the discomfort of breathing in the closed system of the flexible hose 12 and the breathing mask 10. At least one sensor is continuously active to detect occurrence of trigger even including occurrence of snore or sleep apnea, to ensure that trigger events don't go unnoticed at any time.

FIG.6 shows an embodiment of the apparatus of FIG.5.A, wherein the headgear comprises an inflatable headgear 1, and the apparatus further comprises a tube 2 connecting at least one air inflator 3 to the inflatable headgear 1, wherein the inflatable headgear 1 is configured to inflate and expand, and deflate and collapse, at least one release valve 5 configured to deflate the inflatable headgear 1, wherein the control unit 6 is configured to be in communication with the air inflator 3 to actuate the air inflator 3 to inflate the inflatable headgear 1, and the control unit 6 is configured to be in communication with the release valve 5 to close the release valve 5 during the inflation of the inflatable headgear 1 and open the release valve 5 during the deflation of the inflatable headgear 1, wherein the processing unit 7 is configured to transmit control commands to the control unit 6 to turn on and off the air inflator 3 and open and close the release valve 5.

As previously explained, inflatable headgear 1 may comprise an inflatable strap assembly comprising at least one inflatable strap. The different embodiments and configurations of the inflatable strap assembly are also same as previously explained. As explained before, the inflatable strap assembly is configured to extend to the sides and the back of the head of the user. The inflatable headgear 1 may also be configured to extend to a forehead, crown of the head, or the neck of a user. At least one strap that forms the inflatable headgear 1 may comprise at least one inflatable chamber (not shown) positioned inside a strap, where the strap can be larger than the inflatable chamber in width or made of stretchable material to allow for the expansion of the inflatable chamber. The inflatable chamber may be longitudinal and have the shape of a tube. The inflatable chamber can extend to a part or the entirety of the length of the strap. The inflatable strap can be made of any stretchable or non-searchable material, including but not limited to plastic and fabric. The inflatable strap can further comprise an inflatable chamber, where the inflatable chamber is the inflatable strap, and being used instead of or in place of the inflatable strap. The tube 2 connecting the inflatable headgear 1 to the air inflator 3 can be connected to the inflatable headgear 1 anywhere on the inflatable headgear 1 including the back or the front of the inflatable headgear 1. The inflatable headgear 1 comprises an inflatable strap assembly, wherein the inflatable strap assembly comprises of at least one inflatable strap, the inflatable strap assembly may further comprise at least one non-inflatable strap. The inflatable strap may further be combined with non-inflatable straps comprising a strap with inflatable and non-inflatable sections.

The inflation and deflation of the inflatable headgear may also be same as previously explained, wherein the inflation and the expansion of one or more of the inflatable straps of the inflatable strap assembly of the inflatable headgear 1 cause the head of a user to move. The inflatable headgear 1 is configured to inflate and expand, and deflate and collapse, while supporting the head of a user. An inflation and deflation cycle comprises an inflation of the inflatable headgear 1 by the air inflator 3 when actuated by the control unit 6 upon detection of a trigger event, followed by a deflation of the inflatable headgear 1 through the release valve 5 when the control unit 6 signals the opening of the release valve 5. The inflation deflation cycle can comprise the inflatable headgear 1 starting from an initial position of deflation and returning back to the initial position after a cycle of inflation and deflation. The deflation following the inflation can be immediate or can include a time delay. The inflation and deflation of the inflatable headgear 1 each may also occur in multiple steps of inflations and deflations. The inflation and deflation cycle may be configured by timing where in response to the trigger event, the control unit 6 may be configured to actuate the air inflator 3 on with an inflating time and subsequently off with a deflating time, wherein the inflating time and deflating time comprise an inflation and deflation cycle. The inflation and deflation cycle may further be configured by pressure, wherein in response to the trigger event, the control unit 6 is configured to actuate the air inflator 3 on to inflate the inflatable headgear 1 until the control unit 6 reaches a pre-determined pressure and subsequently off until the control unit 6 reaches a deflation pressure, wherein the inflation and deflation comprise a cycle. The inflation of the inflatable headgear 1 may be configured by speed, wherein in the control unit 6 is configured to control a speed at which the air inflator 3 inflates the inflatable headgear 1 at a pre-determined inflation rate to provide different inflation patterns. The inflation of the inflatable headgear 1 may further be configured by size of the expansion of the inflatable headgear 1, wherein the control unit 6 is configured to actuate the air inflator 3 on to inflate the inflatable headgear 1 until the inflatable headgear 1 reaches a predetermined inflation size. The control unit 6 may further be configured to initiate inflation and deflation cycles based on a predetermined time interval, independent of the trigger event.

In accordance with another aspect of the invention the inflatable headgear 1 may further comprise a chin or jaw strap (as shown in FIG.3, but not shown in FIG.6) as part of the inflatable strap assembly. The sections of the chin or jaw strap that extend to the sides of the face, the jaw and the chin of a user may be configured not to be inflatable and function to hold the chin or jaw in place and pull the chin inwards. The chin or jaw strap may be configured to look and function similar to conventional jaw straps or chin straps used to stop and prevent snore and sleep apnea.

In accordance with one aspect of the inventio, upon the detection of the trigger event, the control unit actuates the air inflator on, inflating the inflatable headgear. The control unit 6 is further configured to close the release valve 5 upon detection of the trigger event. The inflation is followed by a deflation as the control unit 6 turns off the air inflator 3 and opens the release valve 5 allowing for deflation of the inflatable headgear 1 and its returning to the initial deflated position. The trigger event may be related to snore or sleep apnea. Examples of the trigger event would be the sound of snore detected by an audio sensor of microphone or detection of sleep apnea from the pattern of breathing indicating occurrence of sleep apnea detected by at least one appropriate sensor that may be positioned inside the breathing mask 10 or at the other end of the flexible hose 12, or anywhere on or off the apparatus. An example may be sound of snore detected by an audio processor, which triggers the inflation of the inflatable headgear 1 followed by its deflation. If the snore or sleep apnea is not stopped, a trigger event will be detected, and an inflation and deflation cycle will take place. The inflation and deflation cycles will continue until the processing unit identifies the cessation of the snore, as long as the processing unit 7 detects a trigger event. The inflation and deflation of the inflatable headgear 1 move the head of a user and may cause the stopping of the snore. This function of the apparatus, in this example, may enabled the stopping is a snore and preventing it from transforming into sleep apnea, and therefore preventing the need to use administration of positive air pressure through the positive airway pressure device 11.

In accordance with yet another aspect of the invention, in response to a trigger event, the control unit 6 may be configured to actuate the air inflator 3 on to inflate the inflatable headgear 1, and close the release valve 5, and also actuate the positive airway pressure device 11 to initiate the administration of positive air pressure through the breathing mask 10, followed by the control unit 6 actuating the active atmospheric or ambient valve 15 to close. The increase of the positive air pressure can take place in instalments and multiple steps as explained previously. The control unit 6 may be configured to actuate the positive airway pressure device 11 to start the administration of positive air pressure followed by the control unit 6 closing the active atmospheric or ambient valve 15 after a predetermined time interval from the detection of the trigger event, while the control unit 6 may be configured to actuate the air inflator 3 and close the release valve 5 immediately upon the detection of a trigger event by the processing unit 7. This will create a delay time between the actuation of the air inflator 3 and the actuation of the positive airway pressure device 11, during which the movements cause by the inflation or the inflation and deflation cycles of the inflatable headgear 1 may stop the snore or sleep apnea and therefore prevent the need of administration of positive air pressure by the positive airway pressure device 11.

In one aspect of the invention, the breathing mask 10 can be attached to and held in place by a non-inflatable headgear (not shown), to make the use of the breathing mask 10 and the inflatable headgear 1 independent of each other. For this embodiment, the apparatus will be configured so that the air inflator 3 and the positive airway pressure device 11 can each be turned off when the user wants to use only one of the two main functions of the apparatus, either the movement by the inflatable headgear 1 or the administration of positive air pressure by the positive airway pressure device 11.

In accordance with yet another aspect of the invention, the active atmospheric or ambient valve, as discussed in the above aspects, can be replaced by a passive atmospheric or ambient valve or any other type of valve that would enable the intended function. In case of a passive valve, the valve will be a normally open valve when there is no administration of positive air pressure. The valve closes as the result of the pressure buildup in the mask when the administration of positive air pressure begins, and is opened again, when the positive air pressure device stops administration of positive air pressure and the pressure in the breathing mask is dropped to the ambient pressure.

## Claims

1. An apparatus comprising:
a breathing mask;
a positive airway pressure device;
a displacement component configured to inflate and expand and deflate and collapse, causing the head of a user to move when worn around the head of the user;
a tube connecting at least one air inflator to the displacement component, wherein the displacement component is configured to inflate and expand, and deflate and collapse;
at least one sensor for detecting a trigger event;
at least one release valve configured to deflate the displacement component;
a flexible hose connecting the breathing mask to the positive airway pressure device;
a control unit in communication with the air inflator to actuate the air inflator on and off and the control unit further in communication with the release valve to close the release valve during the inflation of the displacement component and open the release valve during the deflation of the displacement component, and the control unit is configured to be in communication with the positive airway pressure device;
a processing unit in communication with the control unit, wherein the processing unit is configured to detect the trigger event and transmit control commands to the control unit to trigger actuation of at least one of the air inflator or the positive airway pressure device.

2. The apparatus of claim 1, wherein upon the detection of the trigger event the control unit actuates the air inflator on, inflating the displacement component.

3. The apparatus of claim 1, wherein upon detection of the trigger event, the control unit actuates the positive airway pressure device to at least one of:
initiate the administration of positive air pressure; or,
increase the pressure of the administrated positive air pressure;
wherein the positive air pressure is administered through the breathing mask.

4. The apparatus of claim 3, wherein the control unit is configured to increase the positive air pressure in instalments.

5. The apparatus of claim 1, wherein after a predetermined time during which the processing unit does not detect the trigger event, the control unit is configured to reduce the pressure of or stop the administration of positive air pressure administrated by the positive airway pressure device.

6. The apparatus of claim 5, wherein the control unit is configured to reduce the positive air pressure in instalments.

7. The apparatus of claim 1, wherein at least one sensor is continuously active to detect the occurrence of both snore and apnea.

8. The apparatus of claim 1, wherein in response to the trigger event, the control unit is configured to actuate the air inflator and the positive airway pressure device, wherein the air inflator inflates the displacement component and the positive airway pressure device initiates administration of or increases the pressure of positive air pressure.

9. The apparatus of claim 1, wherein the control unit is configured to:
actuate the air inflator on in response to the trigger event; and,
actuate the positive airway pressure device to start the administration of, or increase the pressure of, positive air pressure, after a predetermined time interval from the detection of the trigger event.

10. The apparatus of claim 1, wherein the control unit is configured to initiate inflation and deflation cycles of the displacement component at predetermined intervals for a duration of the time that the presence of the trigger event is detected.

11. The apparatus of claim 1, wherein the displacement component is configured to interrupt snore and apnea through the movement of the head of the user, wherein the positive airway pressure device is configured to snore and apnea through the use of pressurized air, wherein the movement of the head and pressurized air are used in different orders or in parallel and combination to interrupt or reduce snore or apnea of the user.

12. The apparatus of claim 1, where in use of movement of the head prior to and in combination with the use of pressurized air allows for administration of pressurized air at a lower pressure and for shorter time intervals due to the partial or complete interruption of snore or apnea by the movement of the head caused by the displacement component.

13. The apparatus of claim 1, wherein the trigger event comprises at least one of:
detection of a snore;
detection of an apnea;
identification of a snore or apnea;
identification of a snore or apnea source; or
a prediction of occurrence of snore or apnea.

14. The apparatus of claim 1, wherein the displacement component comprises an inflatable headgear worn around the head of the user, wherein an inflation and deflation cycle comprises an inflation of the inflatable headgear from an initial deflated position, followed by a deflation of the inflatable headgear back to the original deflated position.

15. The apparatus of claim 14, wherein the inflatable headgear is configured to hold the breathing mask in place.

## Patentansprüche

1. Vorrichtung, umfassend:
eine Atemmaske;
ein Gerät für positiven Atemwegsdruck;
eine Verlagerungskomponente, die dazu konfiguriert ist, sich aufzublasen und auszudehnen und Luft abzulassen und zusammenzufallen, wodurch bewirkt wird, dass sich der Kopf eines Benutzers bewegt, wenn sie um den Kopf des Benutzers getragen wird;
eine Röhre, die mindestens eine Luftaufblasvorrichtung mit der Verlagerungskomponente verbindet, wobei die Verlagerungskomponente dazu konfiguriert ist, sich aufzublasen und auszudehnen und Luft abzulassen und zusammenzufallen;
mindestens einen Sensor zum Erkennen eines Auslöseereignisses;
mindestens ein Ablassventil, das dazu konfiguriert ist, die Luft aus der Verlagerungskomponente abzulassen;
einen flexiblen Schlauch, der die Atemmaske mit dem Gerät für positiven Atemwegsdruck verbindet;
eine Steuereinheit, die mit der Luftaufblasvorrichtung in Verbindung steht, um die Luftaufblasvorrichtung durch Betätigen ein- und auszuschalten, und wobei die Steuereinheit ferner mit dem Ablassventil in Verbindung steht, um das Ablassventil während des Aufblasens der Verlagerungskomponente zu schließen und das Ablassventil während des Luftablassens der Verlagerungskomponente zu öffnen, und wobei die Steuereinheit dazu konfiguriert ist, mit dem Gerät für positiven Atemwegsdruck in Verbindung zu stehen;
eine Verarbeitungseinheit, die mit der Steuereinheit in Verbindung steht, wobei die Verarbeitungseinheit dazu konfiguriert ist, das Auslöseereignis zu erkennen und Steuerbefehle an die Steuereinheit zu übermitteln, um die Betätigung von mindestens der Luftaufblasvorrichtung oder dem Gerät für positiven Atemwegsdruck auszulösen.

2. Vorrichtung nach Anspruch 1, wobei die Steuereinheit bei Erkennung des Auslöseereignisses die Luftaufblasvorrichtung durch Betätigen einschaltet und die Verlagerungskomponente aufbläst.

3. Vorrichtung nach Anspruch 1, wobei die Steuereinheit bei Erkennung des Auslöseereignisses das Gerät für den positiven Atemwegsdruck betätigt, um mindestens:
die Verabreichung von positivem Luftdruck einzuleiten; oder,
den Druck des verabreichten positiven Luftdrucks zu erhöhen;
wobei der positive Luftdruck durch die Atemmaske verabreicht wird.

4. Vorrichtung nach Anspruch 3, wobei die Steuereinheit dazu konfiguriert ist, den positiven Luftdruck schrittweise zu erhöhen.

5. Vorrichtung nach Anspruch 1, wobei die Steuereinheit dazu konfiguriert ist, nach einer vorbestimmten Zeit, in der die Verarbeitungseinheit das Auslöseereignis nicht erkennt, den Druck des von dem Gerät für positiven Atemwegsdruck verabreichten positiven Luftdrucks zu reduzieren oder die Verabreichung zu stoppen.

6. Vorrichtung nach Anspruch 5, wobei die Steuereinheit dazu konfiguriert ist, den positiven Luftdruck schrittweise zu reduzieren.

7. Vorrichtung nach Anspruch 1, wobei mindestens ein Sensor kontinuierlich aktiv ist, um das Auftreten sowohl von Schnarchen als auch von Apnoe zu erkennen.

8. Vorrichtung nach Anspruch 1, wobei die Steuereinheit dazu konfiguriert ist, als Reaktion auf das Auslöseereignis die Luftaufblasvorrichtung und das Gerät für positiven Atemwegsdruck zu betätigen, wobei die Luftaufblasvorrichtung die Verlagerungskomponente aufbläst und das Gerät für positiven Atemwegsdruck die Verabreichung von positivem Luftdruck einleitet oder den Druck erhöht.

9. Vorrichtung nach Anspruch 1, wobei die Steuereinheit dazu konfiguriert ist:
die Luftaufblasvorrichtung als Reaktion auf das Auslöseereignis durch Betätigen einzuschalten; und,
das Gerät für den positiven Atemwegsdruck zu betätigen, um nach einem vorbestimmten Zeitintervall ab dem Erkennen des Auslöseereignisses die Verabreichung von positivem Luftdruck zu starten oder den Druck zu erhöhen.

10. Vorrichtung nach Anspruch 1, wobei die Steuereinheit dazu konfiguriert ist, für eine Dauer der Zeit, in der das Vorhandensein des Auslöseereignisses erkannt wird, in vorgegebenen Intervallen Aufblas- und Luftablasszyklen der Verlagerungskomponente einzuleiten.

11. Vorrichtung nach Anspruch 1, wobei die Verlagerungskomponente dazu konfiguriert ist, das Schnarchen und die Apnoe durch die Bewegung des Kopfes des Benutzers zu unterbrechen, wobei das Gerät für den positiven Atemwegsdruck dazu konfiguriert ist, das Schnarchen und die Apnoe durch die Verwendung von Druckluft zu unterbrechen, wobei die Bewegung des Kopfes und die Druckluft in verschiedenen Reihenfolgen oder parallel und in Kombination verwendet werden, um das Schnarchen oder die Apnoe des Benutzers zu unterbrechen oder zu reduzieren.

12. Vorrichtung nach Anspruch 1, wobei die Verwendung der Kopfbewegung vor und in Kombination mit der Verwendung von Druckluft die Verabreichung von Druckluft bei einem niedrigeren Druck und für kürzere Zeitintervalle aufgrund der teilweisen oder vollständigen Unterbrechung des Schnarchens oder der Apnoe durch die von der Verlagerungskomponente bewirkte Kopfbewegung ermöglicht.

13. Vorrichtung nach Anspruch 1, wobei das Auslöseereignis mindestens eines umfasst von:
Erkennung eines Schnarchens;
Erkennung einer Apnoe;
Identifizierung eines Schnarchens oder einer Apnoe;
Identifizierung einer Schnarch- oder Apnoequelle; oder
eine Vorhersage des Auftretens von Schnarchen oder Apnoe.

14. Vorrichtung nach Anspruch 1, wobei die Verlagerungskomponente eine aufblasbare Kopfbedeckung umfasst, die um den Kopf des Benutzers getragen wird, wobei ein Aufblas- und Luftablasszyklus ein Aufblasen der aufblasbaren Kopfbedeckung aus einer anfänglichen abgelassenen Position, gefolgt von einem Luftablassen der aufblasbaren Kopfbedeckung zurück in die ursprüngliche abgelassene Position umfasst.

15. Vorrichtung nach Anspruch 14, wobei die aufblasbare Kopfbedeckung dazu konfiguriert ist, die Atemmaske festzuhalten.

## Revendications

1. Appareil comprenant :
un masque respiratoire ;
un dispositif de ventilation en pression positive ;
un composant de déplacement configuré pour gonfler et augmenter de volume et dégonfler et diminuer de volume, entraînant un mouvement de la tête d'un utilisateur lorsqu'il est porté au autour de la tête de l'utilisateur ;
un tube reliant au moins un dispositif de gonflage pneumatique au composant de déplacement, le composant de déplacement étant configuré pour gonfler et augmenter de volume, et dégonfler et diminuer de volume ;
au moins un capteur destiné à détecter un événement déclencheur ;
au moins une soupape de détente configurée pour dégonfler le composant de déplacement ;
un tuyau flexible reliant le masque respiratoire au dispositif de ventilation en pression positive ;
une unité de commande en communication avec le dispositif de gonflage pneumatique pour mettre en marche et arrêter le dispositif de gonflage pneumatique et l'unité de commande étant en outre en communication avec la soupape de détente pour fermer la soupape de détente pendant le gonflement du composant de déplacement et ouvrir la soupape de détente pendant le dégonflement du composant de déplacement, et l'unité de commande étant configurée pour être en communication avec le dispositif de ventilation en pression positive ;
une unité de traitement en communication avec l'unité de commande, l'unité de traitement étant configurée pour détecter l'évènement déclencheur et transmettre à l'unité de commande des ordres de commande pour déclencher l'actionnement d'au moins un parmi le dispositif de gonflage pneumatique ou le dispositif de ventilation en pression positive.

2. Appareil selon la revendication 1, dans lequel lors de la détection de l'évènement déclencheur l'unité de commande met en marche le dispositif de gonflage pneumatique, faisant gonfler le composant de déplacement.

3. Appareil selon la revendication 1, dans lequel lors de la détection de l'évènement déclencheur l'unité de commande amène le dispositif de ventilation en pression positive à au moins un parmi :
commencer l'administration de pression d'air positive ; ou
augmenter la pression de la pression d'air positive administrée :
la pression d'air positive étant administrée via le masque respiratoire.

4. Appareil selon la revendication 3, dans lequel l'unité de commande est configurée pour augmenter la pression d'air positive par incréments.

5. Appareil selon la revendication 1, dans lequel après un temps préétabli pendant lequel l'unité de traitement ne détecte pas l'évènement déclencheur, l'unité de commande est configurée pour réduire la pression ou arrêter l'administration de pression d'air positive administrée par le dispositif de ventilation en pression positive.

6. Appareil selon la revendication 5, dans lequel l'unité de commande est configurée pour réduire la pression d'air positive par décréments.

7. Appareil selon la revendication 1, dans lequel au moins un capteur est actif en continu pour détecter l'occurrence à la fois de ronflement et d'apnée.

8. Appareil selon la revendication 1, dans lequel en réponse à l'évènement déclencheur, l'unité de commande est configurée pour actionner le dispositif de gonflage pneumatique et le dispositif de ventilation en pression positive, le dispositif de gonflage pneumatique faisant gonfler le composant de déplacement et le dispositif de ventilation en pression positive commençant l'administration de, ou augmentant la pression de, pression d'air positive.

9. Appareil selon la revendication 1, dans lequel l'unité de commande est configurée pour :
mettre en marche le dispositif de gonflage pneumatique en réponse à l'évènement déclencheur ; et,
amener le dispositif de ventilation en pression positive à commencer l'administration de, ou augmenter la pression de, pression d'air positive, après un intervalle de temps préétabli à partir de la détection de l'évènement déclencheur.

10. Appareil selon la revendication 1, dans lequel l'unité de commande est configurée pour commencer des cycles de gonflement et dégonflement du composant de déplacement à intervalles préétablis, pendant une durée de la période où la présence de l'évènement déclencheur est détectée.

11. Appareil selon la revendication 1, dans lequel le composant de déplacement est configuré pour interrompre le ronflement et l'apnée par le mouvement de la tête de l'utilisateur, le dispositif de ventilation en pression positive étant configuré pour le ronflement ou l'apnée par l'utilisation d'air sous pression, le mouvement de la tête et l'air sous pression étant utilisés en divers ordres ou en parallèle et combinaison pour interrompre ou réduire le ronflement ou l'apnée de l'utilisateur.

12. Appareil selon la revendication 1, dans lequel l'utilisation de mouvement de la tête avant et en combinaison avec l'utilisation d'air sous pression permet l'administration d'air sous pression sous une pression plus basse et pendant des intervalles de temps plus courts grâce à l'interruption partielle ou complète de ronflement ou d'apnée par le mouvement de la tête causé par le composant de déplacement.

13. Appareil selon la revendication 1, dans lequel l'évènement déclencheur comprend au moins une parmi :
la détection d'un ronflement ;
la détection d'une apnée ;
l'identification d'un ronflement ou d'une apnée ;
l'identification d'une source de ronflement ou d'apnée ; ou
une prédiction d'occurrence de ronflement ou d'apnée.

14. Appareil selon la revendication 1, dans lequel le composant de déplacement comprend un serre-tête gonflable porté autour de la tête de l'utilisateur, un cycle de gonflement et dégonflement comprenant un gonflement du serre-tête gonflable à partir d'une position dégonflée initiale, suivi d'un dégonflement du serre-tête gonflable reprenant la position dégonflée initiale.

15. Appareil selon la revendication 14, dans lequel le serre-tête gonflable est configuré pour maintenir en place le masque respiratoire.
